# FASCICULE DE BREVET EUROPEEN

(11) **EP 0 634 655 B1**
(45) Date de publication et mention de la délivrance du brevet: **01.09.1999**
(21) Numéro de dépôt: 94420201.9
(22) Date de dépôt: 12.07.1994
(51) Int. Cl.: G01N 33/46, G01N 3/40, G01N 27/04

(54) **Appareillage pour contrôler l'état de dégradation de structure en bois, poteaux notamment**
Vorrichtung zur Prüfung des Schädigungsgrades von Holzstrukturen, insbesondere von Holzmasten
Device for testing the degrading of wooden structures, in particular of poles

(30) Priorité: 13.07.1993 FR 9308848
(43) Date de publication de la demande: 18.01.1995
(73) Titulaire: Sandoz, Jean-Luc, 1110 Morges (CH)
(72) Inventeur: Sandoz, Jean-Luc, 1110 Morges (CH)
(74) Mandataire: Vuillermoz, Bruno

(56) Documents cités:
- DE-A- 1 648 513
- DE-A- 2 919 541
- DE-A- 3 501 841
- DE-A- 4 004 242
- DE-A- 4 122 494
- DE-B- 1 274 818
- DE-U- 9 101 397
- US-A- 4 249 414

## Description

La présente invention a trait à un appareillage, permettant de contrôler, de manière simple, efficace et rapide l'état physique de l'arbre, du bois et de structures en bois, et plus particulièrement de poteaux (poteaux de lignes téléphone, poteaux électriques..), intégrant des variations de densité et soumis dans le temps à des risques de dégradations biologiques affaiblissant leurs propriétés initiales.

Il existe par exemple dans le monde des millions de poteaux en bois en service (10 millions en France, 200 millions aux Etats-Unis par exemple), qu'il faut périodiquement contrôler pour assurer la maintenance des lignes, telles que par exemple lignes de téléphone, lignes électriques, la principale différence entre ces applications résidant dans le diamètre des poteaux inférieur pour les lignes de téléphone (de l'ordre de 17 à 20 cm) à celui des lignes électriques (qui est de l'ordre de 20 à 40 cm).

L'un des principaux problèmes qui se pose avec de tels éléments est celui de leur contrôle périodique pour vérifier leur état afin de décider des opérations d'entretien et/ou de remplacement si cela est nécessaire, par exemple s'ils présentent un risque de rupture, inadmissible compte tenu du fait que, du moins en ce qui concerne les poteaux de lignes téléphoniques, le personnel intervient très souvent en escaladant directement le poteau et que, chaque année, il se produit des accidents parfois mortels du fait de la fragilité mécanique de certains supports.

Actuellement, la solution encore couramment utilisée pour réaliser un tel contrôle, fait appel très souvent à une méthode empirique basée sur l'oreille humaine, le contrôleur donnant quelques coups de marteau sur le poteau et, d'après la réponse acoustique du bois déterminant, grâce à son expérience personnelle, un diagnostic sur l'état du poteau.

De nombreuses propositions ont été faites à ce jour pour réaliser un tel contrôle au moyen d'un appareillage de mesure, tel que cela ressort notamment des brevets US 2,389,030, 4,343,179 et 4 249 414.

D'une manière générale, les solutions proposées selon ces documents consistent à réaliser une mesure de dureté locale. Dans le cas du premier document, on mesure la résistance au vissage dans le bois d'une mèche métallique, dans le second, on provoque une pénétration linéaire d'une "pointe" par un système dynamométrique et, enfin dans le dernier, on mesure la dureté locale de la zone extérieure afin de déterminer si le poteau présente les caractéristiques d'être dur en surface, et par suite d'empêcher l'ancrage des griffes d'un technicien pour l'ascension au moyen d'un ensemble de mesure qui pénètre en biais à l'intérieur du pourtour en formant par rapport à la surface de ce dernier, un angle de quelques degrés.

De telles mesures de la dureté manquent de précision et de fiabilité par le fait qu'elles n'excluent pas la pénétration de l'élément dans une fissure de séchage du poteau. Par ailleurs, elles sont dépendantes d'un effort constant qui conduit donc à une pénétration variable et à un diagnostic qui n'est donc jamais réalisé au niveau des mêmes coordonnées géographiques.

De plus, les solutions décrites dans ces documents sont très sensibles aux variations de diamètre, la pénétration de la mèche ou de la pointe n'étant pas toujours réalisée dans l'axe diamétral, ce qui entraîne une mesure en dehors de l'axe radial naturel des bois, et constitue donc une imprécision supplémentaire.

Enfin, tous les appareillages proposés à ce jour prennent en compte un seul facteur permettant de contrôler la résistance résiduelle du bois, à savoir celui de sa dureté et ne prennent pas en compte un facteur qui est celui de l'hygroscopie pouvant produire des dégâts par pourriture du poteau, et l'affaiblir en son centre sans donner aucun signal de faiblesse de l'extérieur. En effet, comme les poteaux sont traités en autoclave, la couche externe (pouvant aller de 2 cm pour les sapins épicéa à 5 cm pour les pins) est plus résistante dans le temps. Une simple mesure de dureté ne permet pas de détecter si des dégâts interviennent par le coeur du poteau, et l'affaiblissent en son centre, par exemple par suite de pourritures.

Il a également été proposé, ainsi que cela ressort notamment du DE-A-2 919 541, de réaliser la mesure de l'humidité dans un matériau hygroscopique, et plus particulièrement le bois, au moyen d'électrodes pénétrantes fixées sur la tête d'une massette permettant leur mise en position et/ou positionnées sur la tête d'un marteau permettant également par sa masse de faire pénétrer ces électrodes dans le matériau à mesurer.

Une telle mesure a pour but de déterminer la valeur exacte de l'hygroscopie du bois et non pas de donner une indication en fonction de différents seuils prédéterminés, au-delà desquels le niveau d'humidité est susceptible d'entraîner la formation de champignons en activité, entraînant un risque de dégradation interne du bois, ou en revanche, si le taux mesuré est inférieur au seuil critique, de pouvoir assurer qu'il n'y a pas de risque d'activité biologique et que le bois est donc sain.

Or on a trouvé, et c'est ce qui fait l'objet de la présente invention, un appareillage permettant de réaliser aisément et de manière fiable l'établissement d'un diagnostic de l'état du bois, des poteaux ou structures similaires. Par ailleurs, l'appareillage conforme à l'invention autorise une optimisation des interventions et des délais de changement en permettant l'accumulation de données statistiques fiables et précises sur l'état physique et mécanique des poteaux ou autres structures en bois.

D'une manière générale, l'appareillage conforme à l'invention permettant l'établissement d'un diagnostic de l'état de l'arbre, du bois, et de structures en bois, et plus particulièrement des poteaux ou structures similaires, afin d'en déterminer les caractéristiques mécaniques en vue d'effectuer une sélection d'arbres vivants ou de repérer des poteaux affaiblis ou dangereux à ascensionner pour en assurer leur entretien et leur maintenance, comporte des moyens permettant une mesure de la dureté de la structure en bois par pénétration en profondeur dans son épaisseur d'un élément de mesure, caractérisé en ce que :
- l'élément de mesure de dureté est constitué par un ensemble comportant deux pointes, actionnées simultanément et pénétrant par pression dans la structure de part et d'autre d'un entraxe rigide de mesure, et ce sur une profondeur prédéterminée, constante, l'effort réel de pénétration étant mesuré par un capteur de force ;
- les dites pointes sont associées à des moyens permettant simultanément à cette mesure de dureté, d'effectuer une mesure de l'hygroscopie du bois en profondeur constante afin d'évaluer les risques de dégradation biologique interne ;
- l'ensemble est géré par un logiciel traitant les informations obtenues pour donner un résultat instantané concernant la résistance de la structure.

De préférence, dans l'appareillage selon l'invention, la mesure de dureté du bois et d'hygroscopie sont réalisées le plus près possible du niveau de la ligne de sol, les pointes permettant de mesurer la dureté locale étant isolées sur leur longueur et constituant des électrodes permettant de réaliser un contrôle de l'humidité selon la méthode résistive (la résistivité du bois étant inversement proportionnelle à la teneur en eau).

Selon une forme de réalisation l'appareillage selon l'invention, outre sa simplicité, permet de réaliser des mesures très précises et d'établir un diagnostic fiable, et ce sur des éléments en bois pouvant avoir des diamètres variables.

D'une manière générale, un tel appareillage comprend :
- un corps longitudinal mécano-soudé intégrant deux paires de butées de positionnement permettant le montage de l'appareil sur la génératrice longitudinale de la structure (poteau), la fixation étant obtenue par l'intermédiaire de sangles ;
- fixé à la base du corps longitudinal un boîtier comprenant les organes de mesure et d'exploitation constitués par un ensemble comprenant deux pointes associées à un capteur de force et commandées en translation par l'intermédiaire d'un bras de manoeuvre, lesdites pointes constituant des électrodes permettant la mesure de l'hygroscopie en profondeur de la structure en bois.

Dans la suite de la description, l'invention sera décrite plus particulièrement pour le contrôle de poteaux en bois, étant entendu que cela n'est pas limitatif et que, comme indiqué dans le préambule, le procédé et l'appareillage pour sa mise en oeuvre peut être utilisé non seulement pour contrôler des structures autres que les poteaux, mais également être appliqué pour déterminer les caractéristiques d'un arbre vivant (sélection par la densité).

La manière dont est mise en oeuvre l'invention sera cependant mieux comprise grâce à l'exemple de réalisation qui suit, donné à titre indicatif mais non limitatif, et qui est illustré par les schémas annexés dans lesquels :
- la figure 1 est une vue schématique en perspective de l'ensemble d'un appareillage permettant la mise en oeuvre du procédé conforme à l'invention ;
- la figure 2 est une vue de détail en coupe montrant la manière dont est réalisée la pénétration des moyens de mesure à l'intérieur du poteau conformément au procédé selon l'invention ;
- la figure 3 est une vue de détail montrant un mode de réalisation des moyens de fixation dudit appareil de mesure contre la surface du poteau à contrôler.

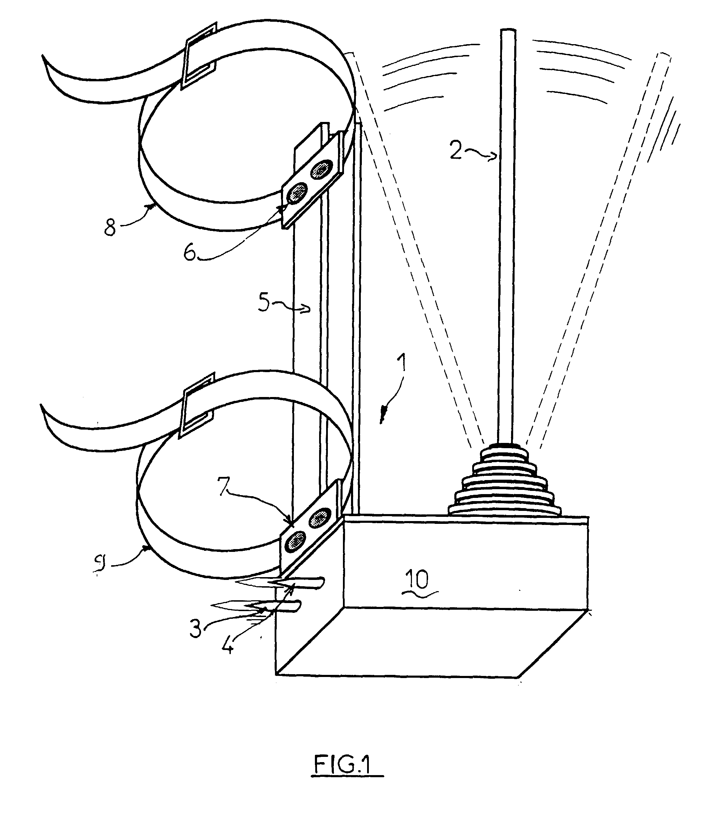

Comme indiqué précédemment, le procédé conforme à l'invention permettant l'établissement d'un diagnostic de l'état physique du bois, ou de structures en bois, plus particulièrement de poteaux, et leur niveau de dégradation éventuel, se caractérise en ce que l'on utilise un appareil de métrologie, illustré à la figure 1 et désigné par la référence générale (1), permettant non seulement d'effectuer la mesure de la dureté, mais également une mesure de l'hygroscopie du bois en profondeur, ces variables permettant, au moyen d'un logiciel de traitement calibré, de donner un résultat instantané et précis à l'utilisateur, sous forme de lampes de couleur (feu vert, sécurité, feu rouge, danger), ou sous forme numérique, transférable à un micro-ordinateur pour statistique de maintenance.

L'appareillage permettant de mettre en oeuvre un tel procédé, est constitué esentiellement par un ensemble susceptible d'être fixé contre la surface du poteau, les moyens de mesure étant disposés à la base de ce dernier et comportant essentiellement un système d'introduction d'effort par bras de levier (2) pour la mesure de dureté, associé à un capteur de force, ainsi qu'une paire d'électrodes (3,4) pour la mesure hygroscopique. L'ensemble est géré par un logiciel, qui exprime un résultat de résistance du poteau basé sur l'étalonnage des deux variables. Le résultat est transmis par l'intermédiaire de lampes, indiquant si le poteau peut être ascensionné, conservé, revisité ou changé sans délai.

Pour réaliser la mesure de l'hygroscopie en profondeur dans le bois, diverses techniques peuvent être envisagées, telles que celles faisant appel aux propriétés isolantes du bois anhydre (état instable) et aux propriétés conductives du bois humidifié. Ces techniques sont dites "résistives", la résistivité du bois étant inversement proportionnelle à sa teneur en eau. Cette technique de mesure sera avantageusement utilisée pour la mise en oeuvre de l'invention, car elle permet d'effectuer une mesure de l'hygroscopie en profondeur, par exemple à 40 mm de la surface, grâce à des électrodes isolées sur leur longueur et qui peuvent constituer les pointes permettant de mesurer la résistance à la pénétration dans le bois.

Eventuellement, il pourrait être envisagé d'autres méthodes connues pour la mesure de l'hygroscopie, par exemple les méthodes capacitives mesurant le déphasage d'une onde haute fréquence après propagation dans la matière. De telles méthodes qui peuvent être plus fines, sont cependant plus délicates à mettre en oeuvre (état de surface et de couplage, sensibilité aux angles de fibres, aux essences ..).

L'ensemble conforme à l'invention permet également d'effectuer la mesure de la dureté locale au niveau de la ligne de sol, et ce en association avec le mécanisme de mesure de l'hygroscopie. Un tel ensemble est constitué par un capteur de force qui mesure la force de pénétration des électrodes dans le bois.

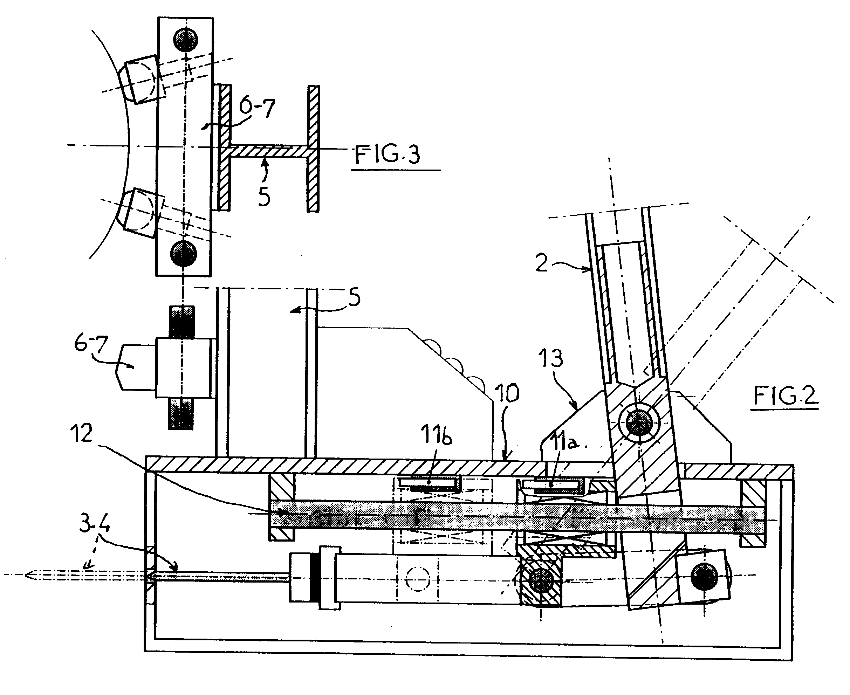

Un tel appareillage peut être réalisé sous la forme illustrée à la figure 1, et est constitué essentiellement de deux parties :
. un corps longitudinal mécano-soudé (5), intégrant deux paires de butées de positionnement (6,7) dont un mode de réalisation détaillé est illustré à la figure 3, garantissant un montage précis de l'appareil sur la génératrice longitudinale du poteau, en sa base directement supérieure au niveau du sol; ce corps (5) se fixe au poteau par l'intermédiaire de sangles (8,9) montées sur cliquet denté, garantissant un bon serrage de l'appareil grâce à la présence de quatre appuis constitués par les deux paires de butées de positionnement (6,7), appuis qui sont eux-mêmes synchronisés sur le détecteur de position de référence (11) quand la tête des électrodes est au contact avec la surface de la génératrice longitudinale du bois, et restant très facile à démonter ;
. à ce corps longitudinal, est associé un boitier (10) comprenant les organes de mesure et d'exploitation, c'est-à-dire les électrodes (3,4), le capteur de force, le dispositif de translation relié au bras de manoeuvre (2) et un ensemble supportant le traitement et l'affichage des données sur lampes de couleur, ainsi que la liaison possible à un micro-ordinateur, et la mémoire pour la gestion du cycle de mesure et la mémorisation d'acquisition des données.

La figure 2 illustre une forme de réalisation concrète de l'ensemble d'un tel boitier (10), qui outre les moyens précités, comporte également des capteurs (11a,11b) de début et fin de course, une alimentation électrique et une chaine de mesure du capteur de force et de la résistivité entre les électrodes. Il supporte également un jeu de lampes permettant l'affichage, en code couleur, de l'état physico-mécanique du poteau.

La pénétration des électrodes à l'intérieur du poteau, est réalisée par la force manuelle de l'utilisateur, la profondeur de pénétration (longueur de l'électrode) pouvant être variable mais généralement calibrée à 40 mm. A titre indicatif, les efforts maximaux nécessaires pour la pénétration des pointes dans un poteau neuf de forte densité, sont d'environ 3000 N par couple d'électrodes de 3 à 3,5 mm de diamètre. Cet effort correspond au pic de pression observé après 40 mm d'enfoncement (pression plus frottement).

Pour garantir l'engagement et le dégagement des électrodes, leur déplacement est réalisé par une translation linéaire du support des électrodes qui est aussi le support du capteur d'effort. Cette translation est provoquée par l'action humaine, et elle est garantie dans le système de guidage mécanique (12) qui est protégé par le boitier (10), et dont l'étanchéité est assurée par un soufflet (13), guidage qui garantit une translation constante dans le temps, notamment au niveau des frottements. Ceci contribue également à la précision et à la fiabilité des performances de l'appareil.

La pression de mesure n'est pas influencée par le diamètre du poteau grâce à l'assurance d'un système de positionneurs alignés sur les électrodes. En conséquence, grâce à l'invention, il est possible de contrôler des poteaux de diamètres différents (des poteaux de téléphone de petits diamètres au poteau de lignes électriques de moyennes à haute tension). Le capteur de force monté derrière la tête des électrodes, mesure l'effort de pénétration desdites électrodes, et ce avec une précision supérieure à 2 %. La qualité de la translation permet une excellente répétitivité des mesures.

Le corps du boitier dudit appareil, intègre le cablage des deux paires d'organes de mesure de façon à les relier à l'unité de gestion. La gestion est réalisée en temps réel, avec transmission des informations, par exemple sur des lampes indiquant l'état instantané du bois ou du poteau, ou encore, par liaison avec un micro-ordinateur capable d'interpréter les mesures réelles effectuées, mémorisables dans l'appareil, pour des études statistiques visant à l'optimisation de la maintenance du matériel visité. Il pourrait être envisagé d'utiliser d'autres moyens de traitement, par exemple avant mémorisation ou par l'intermédiaire des sorties en lignes séries.

Quand les électrodes sont en bout de course (après 40 mm de pénétration par exemple), le capteur transmet l'information et déclenche la saisie des mesures de la dureté par lecture du capteur de force.

L'hygroscopie est quant à elle mesurée en tête des électrodes, ce qui permet de connaitre l'hygroscopie en profondeur du poteau. Le corps des électrodes est isolé de façon à ne pas être perturbé par les hygroscopies de surface.

La mesure de l'hygroscopie est réalisée de manière très précise compte tenu du fait qu'elle est effectuée avec les électrodes agissant selon un plan horizontal dans le poteau encastré dans le sol, ce qui garantit donc une mesure au travers d'une couche de cellules plus importante.

Un tel appareil est de conception simple et facile à mettre en oeuvre, les déplacements des électrodes (3,4) se faisant facilement par le biais des déplacements du bras de levier autour de son articulation ; la course des électrodes est limitée par une chainette d'arrêt ou autres moyens équivalents.

Il convient également de noter que dans le mode de réalisation préférentiel illustré par la figure 1, la manière dont est fixé l'appareillage conforme à l'invention permet d'obtenir une très grande stabilité de fixation sur le poteau grâce à la présence de quatre appuis constants, reproductibles quel que soit le diamètre du poteau et stables quelle que soit sa dureté, la pénétration des éléments de mesure étant par exemple réalisée de manière précise dans l'axe diamétral.

Par rapport aux solutions antérieures, un tel procédé (et appareillage) présente de très nombreux avantages et conduit à une très grande précision de mesure, compte tenu du fait qu'il se base sur la métrologie de deux variables (dureté locale et hygroscopie). Le fait que cette mesure soit effectuée au moyen de deux pointes élimine tous risques de mesure faussée, par exemple par pénétration dans une fissure du poteau, le procédé selon l'invention permettant de mesurer un effort par capteur de force, et ce à une distance constante (celle de l'enfoncement de toutes les pointes) garantit une précision dans la mesure de dureté. Par ailleurs, la mesure en profondeur de l'hygroscopie, permet de déterminer si la structure n'est pas fragilisée par l'action biologique d'un champignon lignivore, cette mesure permettant de mettre en évidence une plage hygroscopique correspondant à la possibilité de présence du parasite. Cette mesure n'a pas pour but de donner une valeur forcément exacte de l'hygroscopie, mais une indication d'ordre biologique, à savoir si le bois contient vraisemblablement un champignon en activité ou s'il est sain.

Enfin, selon l'invention, l'exploitation des résultats est basée sur une analyse électronique et un diagnostic est réalisé sur la base d'un étalonnage en laboratoire, assurant une résistance caractéristique du poteau à chaque couple de données mesurées. L'interprétation, effectuée par la machine qui transpose à l'utilisateur des signaux, par l'intermédiaire de diodes lumineuses traduisant des états différents, par exemple très bon état, bon état, en cours d'affaiblissement et affaibli, très affaibli et dangereux.

Un tel type de diagnostic garantit donc la sécurité d'exploitation du réseau ainsi qu'une meilleure gestion de la maintenance du matériel visité, par l'exploitation par un micro-ordinateur de toutes les données réelles mesurées et stockées temporairement dans l'appareil.

## Revendications

1. Appareillage permettant l'établissement d'un diagnostic de l'état de l'arbre, du bois, et de structures en bois, et plus particulièrement des poteaux ou structures similaires, afin d'en déterminer les caractéristiques mécaniques en vue d'effectuer une sélection d'arbres vivants ou de repérer des poteaux affaiblis ou dangereux à examiner pour en assurer leur entretien et leur maintenance, comportant des moyens permettant une mesure de la dureté de la structure en bois par pénétration en profondeur dans son épaisseur d'un élément de mesure, caractérisé en ce que :
- l'élément de mesure de dureté est constitué par un ensemble comportant deux pointes (3,4), actionnées simultanément et pénétrant par pression dans la structure de part et d'autre d'un entraxe rigide de mesure, et ce sur une profondeur prédéterminée, constante, l'effort réel de pénétration étant mesuré par un capteur de force ;
- lesdites pointes sont associées à des moyens permettant, simultanément à cette mesure de dureté, d'effectuer une mesure de l'hygroscopie du bois en profondeur constante afin d'évaluer les risques de dégradation biologique interne ;
- l'ensemble est géré par un logiciel traitant les informations obtenues pour donner un résultat instantané concernant la résistance de la structure.

2. Appareillage selon la revendication 1, ***caractérisé*** en ce que la mesure de dureté du bois et d'hygroscopie sont réalisées le plus près possible du niveau de la ligne de sol, les pointes permettant de mesurer la dureté locale étant isolées sur leur longueur et constituant des électrodes permettant de réaliser un contrôle de l'humidité selon la méthode résistive, la résistivité du bois étant inversement proportionnelle à la teneur en eau.

3. Appareillage selon l'une des revendications 1 et 2, caractérisé en ce qu'il comprend :
- un corps longitudinal mécano-soudé (5) intégrant deux paires de butées de positionnement (6,7) permettant le montage de l'appareil sur la génératrice longitudinale de la structure, la fixation étant obtenue par l'intermédiaire de sangles (8,9) ;
- fixé à la base du corps longitudinal (5) un boîtier (10) comprenant les organes de mesure et d'exploitation constitués par un ensemble comprenant les deux pointes (3,4) associées à un capteur de force et commandées en translation par l'intermédiaire d'un bras de manoeuvre (2), lesdites pointes (3,4) constituant des électrodes permettant la mesure de l'hygroscopie en profondeur de la structure en bois.

## Claims

1. Device making it possible to establish a diagnosis of the physical state of trees, of wood and of wooden structures, and more particularly of poles or similar structures, so as to determine the mechanical characteristics of them with a view to making a selection of living trees or to identifying weakened or dangerous poles that must be climbed in order to provide upkeep and maintenance on them, comprising means carrying out a measurement of the hardness of the wooden structure by depthwise penetration into its thickness of a measurement element, characterized in that :
_ the hardness measurement element is constituted by an assembly comprising two spikes (3,4) which are simultaneously actuated and penetrate by means of pressure into the structure on either side of a rigid measurement spacing, and to a predetermined and constant depth, the actual penetration force being measured by a force sensor ;
_ said spikes are associated with means allowing, simultaneously with this hardness measurement, to realize a measurement of the moisture content of the wood at a constant depth in order to evaluate the risk of internal biological degradation ;
_ the whole assembly being managed by a software processing the informations obtained in order to give an instantaneous result relating to the strength of the structure.

2. Device as claimed in claim 1, characterized in that the measurement of the hardness of the wood and of the moisture content are carried out as close as possible to ground level, the spikes which enable the local hardness to be measured being insulated over their length and constituting electrodes making it possible to check the moisture content using the resistive method, the resistively of the wood being inversely proportional to the water content.

3. Device as claimed in one of claims 1 and 2, characterized in that it comprises :
_ an all-welded longitudinal body (5) incorporating two pairs of positioning stops (6,7) enabling the apparatus to be fitted along the longitudinal generatrix of the wooden structure, fixing being achieved by means of straps (8,9) ;
_ a case (10) fixed to the base of the longitudinal body (5) and comprising measurement and application members constituted by an assembly comprising said two spikes (3,4) associated with a force sensor and driven translationally by means of an operating arm (2), said spikes (3,4) constituting electrodes enabling the depthwise measurement of the water content of the wooden structure to be measured.

## Patentansprüche

1. Vorrichtung für die Erstellung einer Diagnose über den Zustand von Bäumen, Holz und Holzstrukturen, insbesondere von Masten oder ähnlichen Strukturen, zur Bestimmung der mechanischen Merkmale zur Durchführung einer Selektion von lebenden Bäumen oder zur Kennzeichnung von Masten, die geschwächt oder gefährlich zu prüfen sind, um ihren Unterhalt und ihre Wartung zu gewährleisten, mit Mitteln zur Messung der Härte der Holzstruktur durch Tiefeneindringung eines Meßelements in ihre Dicke, dadurch gekennzeichnet, daß
- das Element zur Härtemessung aus einer Einheit besteht, die zwei Spitzen (3, 4) umfaßt, die gleichzeitig betätigt werden und durch Druck zu beiden Seiten eines starren Meßachsabstandes in die Struktur eindringen, und zwar über eine vorbestimmte konstante Tiefe, wobei die tatsächliche Eindringkraft durch einen Kraftfühler gemessen wird,
- den Spitzen Mittel zugeordnet sind, die gleichzeitig mit der Härtemessung die Durchführung der Messung der Wasseraufnahme des Holzes in konstanter Tiefe gestatten, um die Gefahren der inneren biologischen Beschädigung zu ermitteln,
- die Einheit durch eine Software gesteuert wird, die die erhaltenen Daten verarbeitet, um ein momentanes, die Festigkeit der Struktur betreffendes Ergebnis zu erhalten.

2. Vorrichtung nach Anspruch 1, dadurch gekennzeichnet, daß die Messung der Härte des Holzes und der Wasseraufnahme möglichst nahe bei der Höhe der Bodenlinie stattfindet, wobei die die Messung der örtlichen Härte gestattenden Spitzen auf ihrer Länge isoliert sind und Elektroden bilden, die die Prüfung der Feuchtigkeit mit Hilfe der resistiven Methode gestatten, wobei der spezifische Widerstand des Holzes zum Wassergehalt umgekehrt proportional ist.

3. Vorrichtung nach einem der Ansprüche 1 und 2, dadurch gekennzeichnet, daß sie umfaßt:
- einen maschinengeschweißten Längskörper (5), in den zwei Paare von Positionierungsanschlägen (6, 7) integriert sind, die die Montage der Vorrichtung auf der Längserzeugenden der Struktur gestatten, wobei die Befestigung über Gurte (8, 9) erreicht wird,
- ein an der Basis des Längskörpers (5) befestigtes Gehäuse (10), das die Organe zur Messung und zur Verarbeitung enthält, die aus einer Einheit bestehen, die die beiden Spitzen (3, 4) umfaßt, denen eine Kraftfühler zugeordnet ist und die in Translationsbewegung über einen Betätigungsarm (2) gesteuert werden, wobei die Spitzen (3, 4) Elektroden bilden, die die Messung der Wasseraufnahme in der Tiefe der Holzstruktur gestatten.
